# EUROPÄISCHE PATENTANMELDUNG

(11) **EP 2 926 837 A1**
(43) Veröffentlichungstag der Anmeldung: **07.10.2015**
(21) Anmeldenummer: 15161071.4
(22) Anmeldetag: 26.03.2015
(51) Int. Cl.: A61L 2/18, F16L 33/28, F16L 37/26

(54) **KUPPLUNG**

(30) Priorität: 04.04.2014 DE 102014104848
(71) Anmelder: Schulz GmbH, 58540 Meinerzhagen (DE)
(72) Erfinder: Schulz, Hartmut, 58540 Meinerzhagen (DE)
(74) Vertreter: Patentanwälte Dörner & Kötter PartG mbB

(57) **Zusammenfassung**

Die Erfindung betrifft eine Kupplung (3, 3') zum zeitweisen Anschließen eines Flüssigkeitsschlauches (4, 4') an einen Stutzen (2, 2') eines mit Desinfektions- oder Reinigungsmittel befüllten Behälters (1), die von einem Flanschteil (31, 31') und einem Anschlussstück (32, 32') gebildet ist. Das Flanschteil (31, 31') ist an dem Stutzen (2, 2') angeordnet und das Anschlussstück (32, 32') ist an dem Schlauch (4, 4') angeordnet. An dem Anschlussstück (32, 32') und / oder dem Flanschteil (31, 31') ist eine mechanische Codierung (6, 6') vorgesehen.

## Beschreibung

Die Erfindung betrifft eine Kupplung zum zeitweisen Anschließen eines Flüssigkeitsschlauches an einen Stutzen eines mit Desinfektions- oder Reinigungsmittel befüllten Behälters, die von einem Flanschteil und einem Anschlussstück gebildet ist, wobei das Flanschteil an dem Stutzen angeordnet ist und das Anschlussstück an dem Schlauch angeordnet ist.

Bei der Verwendung von Desinfektions- oder Reinigungsmitteln ist zur Vermeidung von Fehlbedienungen eine große Sorgfaltspflicht gefordert. Insbesondere bei der Mischung von Gebrauchslösungen aus verschiedenen Desinfektionsund/oder Reinigungsmitteln sowie bei der Zubereitung von Desinfektions- oder Reinigungsmittelgebrauchslösungen in Verbindung mit Trinkwassernetzen ist diese Sorgfalt geboten, da durch Mischungen in falschen Verhältnissen oder aufgrund der Abgabe einer zu großen Menge der Desinfektions- und Reinigungsmittel eine Überschreitung von Grenzwerten erfolgen kann, die für die Bedienperson gesundheitsgefährlich sein kann. Für den Fall, dass die überkonzentrierte Gebrauchslösung in das Trinkwasser gelangt, können erhebliche Umweltschäden auftreten.

Um beim Anschließen der die Desinfektions- oder Reinigungsmittel beinhaltenden Behälter an Misch- oder Dosiereinrichtungen Fehlanschlüsse zu vermeiden, sind Codierungen an den Behältern bzw. deren Anschlüssen vorgesehen. Diese sind von computer-lesbaren Codierungen gebildet. Bei diesen kann es sich beispielsweise um Barcodes handeln. Auch die Verwendung von integrierten Computerchips ist möglich. Den bekannten Lösungen ist der für die Identifizierung erforderliche hohe technische Aufwand gemein. Zur einwandfreien Identifizierung ist es nämlich erforderlich, die Codierungen auszulesen, was mit Hilfe von computerunterstützten Lesegeräten erfolgt. Dies hat einen hohen technischen Aufwand zur Folge, der mit hohen Kosten verbunden ist.

Hier will die Erfindung Abhilfe schaffen. Der Erfindung liegt die Aufgabe zugrunde, eine Kupplung zum zeitweisen Anschließen eines Flüssigkeitsschlauches an einen Stutzen eines mit Desinfektions- oder Reinigungsmittel gefüllten Behälters zu schaffen, die einerseits eine Fehlbedienung, insbesondere einen Fehl-Anschluss ausschließt, und andererseits einen geringen technischen Aufwand erfordert. Gemäß der Erfindung wird diese Aufgabe dadurch gelöst, dass an dem Anschlussstück und/oder dem Flanschteil eine mechanische Codierung vorgesehen ist.

Mit der Erfindung ist eine Kupplung zum zeitweisen Anschließen eines Flüssigkeitsschlauchs an einen Stutzen eines mit Desinfektions- oder Reinigungsmittel gefüllten Behälters geschaffen, die eine Fehlbedienung ausschließt. Dies ist durch die mechanische Codierung hervorgerufen, welche lediglich den Anschluss des mit der jeweiligen Codierung korrespondierenden Teils der Kupplung zulässt. Somit ist ein Fehlanschluss ausgeschlossen.

In Weiterbildung der Erfindung ist die mechanische Codierung an dem Anschlussstück von mindestens zwei Stiften gebildet, die mit mindestens zwei Aufnahmen an dem Flanschteil korrespondieren. Durch diese Ausgestaltung ist in einfacher Weise eine zuverlässige Codierung geschaffen. Durch die Korrespondenz zwischen den Stiften des Anschlussstücks und den Aufnahmen des Flanschteils ist bewirkt, dass lediglich bei aufeinander abgestimmten geometrischen Verhältnissen der Stifte und der Aufnahmen ein Anschluss möglich ist. Sobald die Stifte und die Aufnahmen aufgrund unterschiedlicher Abmessungen, Abständen oder dergleichen nicht miteinander korrespondieren, ist ein Anschluss verhindert. Somit ist gleichzeitig ein fehlerhafter Anschluss vermieden.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:
- Figur 1: die schematische Darstellung einer Entnahmestelle mit Mischund Dosiereinrichtung;
- Figur 2: die schematische Darstellung einer Kupplung mit mechanischer Codierung;
- Figur 3: die schematische Darstellung einer zweiten Kupplung mit mechanischer Codierung.

Die als Ausführungsbeispiel gewählte Kupplung dient zum zeitweisen Anschließen eines Flüssigkeitsschlauchs an einen Stutzen eines mit Desinfektions- oder Reinigungsmittel befüllten Behälters. Im Ausführungsbeispiel sind an einer Entnahmestelle zwei Behälter 1,1' vorgesehen, die jeweils mit einem Stutzen 2, 2' versehen sind. An den Stutzen 2, 2' ist jeweils eine Kupplung 3, 3' angeschlossen, die das Ende eines Schlauchs 4, 4' bildet. Die Schläuche 4, 4' sind an eine Misch- und Dosiereinrichtung 5 angeschlossen.

Die Kupplungen 3, 3' sind von einem Flanschteil 31, 31' und einem Anschlussstück 32, 32' gebildet. Die Flanschteile 31, 31' sind an den Stutzen 2, 2' der Behälter 1,1' angeordnet. Die Anschlussstücke 32, 32' sind an die Schläuchen 4, 4' angeschlossen.

Die Kupplungen 3, 3' sind mit einer mechanischen Codierung 6, 6' versehen. Im Ausführungsbeispiel ist an den Flanschteilen 31, 31' und den Anschlussstücken 32, 32' jeweils eine mechanische Codierung 6, 6' vorgesehen. In Abwandlung des Ausführungsbeispiels besteht auch die Möglichkeit, eine mechanische Codierung lediglich an dem Anschlussstück oder an dem Flanschteil vorzusehen.

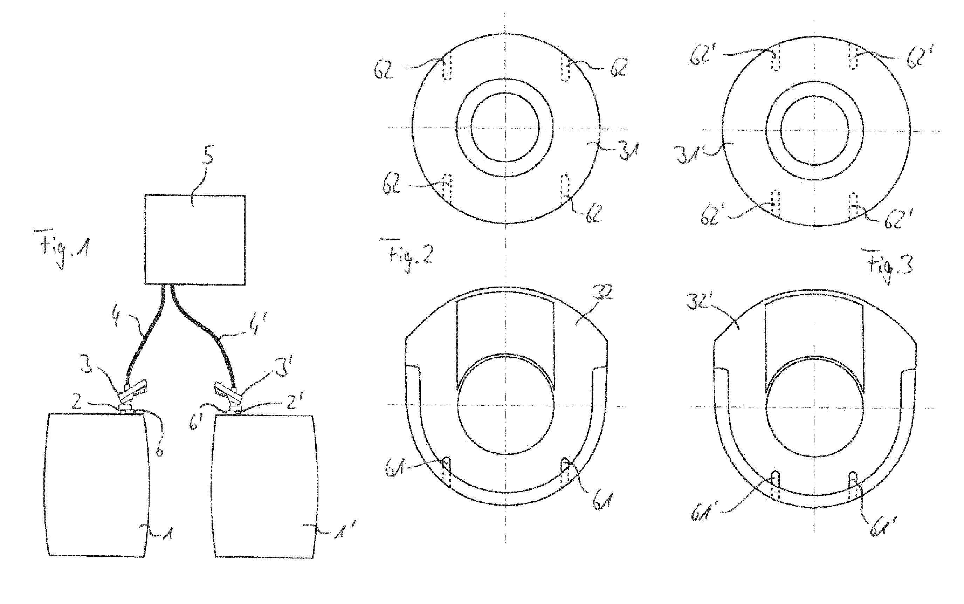

Die mechanische Codierung 6, 6' ist im Ausführungsbeispiel an dem Anschlussstück 32, 32' von zwei Stiften 61, 61' gebildet, die horizontal zueinander beabstandet und zueinander parallel ausgerichtet sind. Die Stifte 61, 61' korrespondieren mit Aufnahmen 62, 62' an den Flanschteilen 31, 31'. Im Ausführungsbeispiel sind an den Flanschteilen 31, 31' jeweils vier Aufnahmen 62, 62' vorgesehen. Dadurch ist die Möglichkeit geschaffen, die Anschlussstücke 32, 32' in zwei unterschiedlichen Positionen, nämlich um 180° verdreht, an den Flanschteilen 31, 31'. anzuordnen, wodurch beim Anschluss auf örtliche Gegebenheiten vor Ort Rücksicht genommen werden kann. So ist vermieden, dass unter schwierigen örtlichen Gegebenheiten für den Anschluss die Behälter 1, 1' gedreht werden müssen, um einen Anschluss zu ermöglichen. Im Ausführungsbeispiel sind jeweils zwei Aufnahmen 62, 62' einander gegenüberliegend an den Flanschteilen 31, 31' angeordnet, In verriegeltem Zustand der Flanschteile 31, 31' mit den Anschlussstücken 32, 32' befinden sich die Stifte 61, 61' in den Aufnahmen 62, 62'. Wie den Figuren 2 und 3 zu entnehmen ist, weisen die Stifte 61 an dem Anschlussstück 32 einen größeren Abstand zueinander auf, als die Stifte 61' des Anschlussstücks 32'. Ebenso verhält es sich mit dem Abstand der Aufnahmen 62, 62' an den Flanschteilen 31, 31'. Aus dem unterschiedlichen Abstand wird unmittelbar erkennbar, dass eine Verbindung des Flanschteils 31 mit dem Anschlussstück 32' ebenso wenig möglich ist, wie der Anschluss des Flanschteils 31' mit dem Anschlussstück 32, da aufgrund der unterschiedlich zueinander beabstandeten Stifte 61, 61' eine Anordnung in den Aufnahmen 62, 62' verhindert ist. Somit besteht einzig die Möglichkeit, das Flanschteil mit dem Anschlussstück 32 zu verbinden und das Flanschteil 31' mit dem Anschlussstück 32 zu verbinden. Eine Fehlbedienung beim Anschließen eines Flüssigkeitsschlauchs an den Stutzen eines mit Desinfektions- oder Reinigungsmittel befüllten Behälters ist somit ausgeschlossen. Aufgrund der allein mechanischen Codierung ist ein Auslesen irgendwelcher elektronischen Codierungen nicht erforderlich, so dass auch keine zusätzlichen Auslesegeräte erforderlich sind. Die erfindungsgemäße mechanische Codierung ist daher preiswert und zugleich außerordentlich zuverlässig.

## Patentansprüche

1. Kupplung zum zeitweisen Anschließen eines Flüssigkeitsschlauches (4, 4') an einen Stutzen (2, 2') eines mit Desinfektions- oder Reinigungsmittel befüllten Behälters (1), die von einem Flanschteil (31, 31') und einem Anschlussstück (32, 32') gebildet ist, wobei das Flanschteil (31, 31') an dem Stutzen (2, 2') angeordnet ist und das Anschlussstück (32, 32') an dem Schlauch (4, 4') angeordnet ist, **dadurch gekennzeichnet, dass** an dem Anschlussstück (32, 32') und / oder dem Flanschteil (31, 31') eine mechanische Codierung (6, 6') vorgesehen ist.

2. Kupplung nach Anspruch 1, **dadurch gekennzeichnet, dass** die mechanische Codierung (6, 6') an dem Anschlussstück (32, 32') von mindestens zwei Stiften (61, 61') gebildet ist, die mit mindestens zwei Aufnahmen (62, 62') an dem Flanschteil (31, 31') korrespondieren.
